# Europäisches Patentamt

# European Patent Office

# Office européen des brevets

(11) Publication number: **0 022 669**
**B2**

(12) **NEW EUROPEAN PATENT SPECIFICATION**

(45) Date of publication of the new patent specification: **06.06.90**

(51) Int. Cl.⁵: **G 01 N 33/80**

(21) Application number: **80302359.7**

(22) Date of filing: **11.07.80**

(54) Method and reagent for blood typing and method for preparing this reagent.

(30) Priority: **13.07.79 US 57481**
**05.10.79 US 82199**
**09.06.80 US 155322**

(43) Date of publication of application:
**21.01.81 Bulletin 81/03**

(45) Publication of the grant of the patent:
**29.01.86 Bulletin 86/05**

(45) Mention of the opposition decision:
**06.06.90 Bulletin 90/23**

(84) Designated Contracting States:
**CH DE FR GB IT LI NL SE**

(56) References cited:
DE-A-2 614 865
DE-A-2 636 616
US-A-3 579 627
US-A-3 880 988

PROC. NATL. ACAD. SCI. USA, Vol. 74, No. 6, June 1977 D.G. ROMANS et al. "Conversion of Incomplete Antibodies to Direct Agglutinins by Mild Reduction: Evidence for Segmental Flexibility within the Fc Fragment of Immunoglobulin G" pages 2531 to 2535

The file contains technical information submitted after the application was filed and not included in this specification

(73) Proprietor: **ORTHO DIAGNOSTIC SYSTEMS INC.**
**One Johnson & Johnson Plaza**
**New Brunswick New Jersey 08933-7003 (US)**

(72) Inventor: **Graham Jr., Henry Alexander**
**RD No.2, Box 74**
**Annandale, New Jersey (US)**
Inventor: **Olekna, David John**
**23, West Street**
**Annandale, New Jersey (US)**
Inventor: **Hawk, Johnna Berenice**
**73 Washington Street**
**Rocky Hill,New Jersey (US)**
Inventor: **Kebles, Diane Barrett**
**561, T Fox Run Road, RD No. 1**
**Stewartsville, New Jersey 08886 (US)**

(74) Representative: **Jones, Alan John et al**
**CARPMAELS & RANSFORD 43 Bloomsbury Square**
**London, WC1A 2RA (GB)**

(56) References cited:
NATURE, Vol. 197, January 1963 B. PIROFSKY et al. "Bivalent Nature of Incomplete Anti-D(Rho)" pages 392 to 393

Biblitheca Haematologica no 38 part 1, 1971. KJG Goldsmith et al pp 138-141

**EP 0 022 669 B2**

(56) References cited:

Transfusion vol. 18no 4 JUL-AUG 1978, R P
Reckel & J Harris pp 387-406

P Schwarz Dissertation, Ruprecht Karl
Universität, Heidelberg 1970 pp 16-17

CRC Handbook Series in clinical lab. science
Section D, Blood banking vol. 1 Cleveland 1970
p 419

## Description

Technical Field

The present invention relates to methods and reagents for use in blood typing and more specifically for the detection of $Rh_o$ (D) antigen.

Background of the Invention

Blood typing, and particularly the determination of the presence or absence of $Rh_o$ (D) antigen, is a routine procedure in modern medicine. Since the discovery of the relationship between Rh factor and disease in the 1930's, there has been an increasing concern to detect Rh incompatibilities between mother and fetus so that such incompatibilities may be treated or avoided in future children. At the present time, many state laws require Rh testing of pregnant women and of infants born of Rh negative women. The introduction of Rh immuno globulin in 1968 allowed for the first time a method of treatment of Rh negative women bearing Rh positive children to prevent disease in later Rh positive children of these women.

These are two types of diagnostic tests currently used for detecting the presence of D antigen. It should be understood, by way of introduction, that IgG antibody to D antigen is a so-called "incomplete" antibody. That is, IgG antibodies with specificity for the D antigen often fail to agglutinate Rh positive red cells suspended in saline. In contrast, Igm antibodies to the D antigen do cause agglutination of Rh positive red cells in saline.

The first of these tests uses IgG antibody as the reagent. Since anti-D IgG will not by itself cause agglutination of Rh positive red blood cells in saline, some potentiating means must be found to cause the agglutination. This potentiation is accomplished by a relatively high concentration of a biological polymer (e.g., nonspecific protein such as albumin) or a synthetic polymer (e.g., polyvinylpyrrolidone or a dextran sulfate). Typically, the concentration of albumin in the final reagent composition is approximately 20 to 25 percent. While this added protein succeeds in potentiating the agglutination of specific IgG anti-D antibodies and red blood cells having D antigen, it also causes nonspecific agglutination of red blood cells having IgG coated on the surface thereof. Such coating occurs, for example, in individuals having such diseases as autoimmune hemolytic anemia or hemolytic disease of the newborn, in which antibody is produced to the patient's own red blood cells and binds thereto. False positive results for presence of Rh antigen will therefore be obtained for these individuals with this test. One significant advantage of this test, however, is its rapidity, since little (if any) incubation is required prior to reading the results. This test may be conducted in either a test tube or a glass slide. A second advantage is the ability to detect certain weak antigens denominated $D^u$, as further described below.

The second current test for detection of D antigen uses IgM antibody instead of IgG antibody. Since IgM is a complete antibody with respect to the D antigen, the addition of a potentiator is not required; typically, the reagent composition contains a total protein concentration of less than about 8 percent by weight, accomplished by adding (e.g.) bovine albumin to the IgM antiserum. This test can be conducted only in a test tube. While this test removes the nonspecific agglutination of IgG coated cells which occurs with the potential IgG test, it also suffers from several disadvantages. The principal disadvantages are that the test requires a fifteen to sixty minute incubation at 37°C, which step is not required by the potentiated IgG test, and also that it requires IgM antibody, which is rare and difficult to obtain. Additionally, it is not possible to run the so-called $D^u$ test in conjuction with this second type of test, since this $D^u$ test uses antibody to IgG as its reagent. This reagent antibody obviously does not react with IgM.

Thus, if one could develop a rapid test for blood group antigens employing IgG antibody in a low protein formulation, a long felt need would be satisfied.

Prior Art

The conversion of incomplete anti-D IgG to a direct saline agglutinin by reductive cleavage of the disulfide bonds has been described in several articles. It was first reported in 1960 by Chan and Deutsch (J. Immunol. *85;* 37—45) and then in 1963 by Pirofsky and Cordova (Nature, *197,* 392—393), but these findings were called into question in 1965 by Mandy, *et al.* (J. Clin. Invest., *44,* 1352—1363). Additional work confirming the conclusions of Pirofsky and Cordova was reported in 1977 by Romans, *et al.,* [Proc. Natl. Acad. Sci. USA, *74* (6), 2531—2535]. These latter authors reported the conversion of not only anti-D IgG but also anti-c, anti-E, and anti-K IgG to direct saline agglutinins. In this article, however agglutination was achieved only in a test tube after a two hour incubation of red blood cells and the reduced IgG. Morever, although this Romans, *et al.,* article teaches that incomplete IgG antibodies may be converted to complete antibodies by reduction, there is no teaching of any specific procedure or reagent (let alone one meeting FDA standards) for use in conducting a typing test. In fact, the article indicates in the last sentence that there may be some question as to whether the reduced antibody will be practical for use as a typing reagent, referring to certain unpublished studies then in progress.

In the report of the combined meeting of the XVII Congress of the International Society of Hermatology and the XV Congress of the International Society of Blood transfusion, July 24—28, 1978, there was published an abstract which appears to disclose some of the work referred to in the Romans, *et al.,* paper. This Laschinger *et al.,* abstract reports "the production and testing of a reliable monospecific anti-D agglutinin with a titer of 16 from a pool of 'incomplete' anti-D sera. Modification of pooled 'incomplete' anti-c produced a direct reagent with a titer of 88". These reporterd titers give no assurance that the materials produced met FDA standards. No information is given in the abstract relating to the method by which these agglutinins were produced, the test method used, or the amount of incubation necessary to achieve agglutination. Moreover the abstract indicates that a usable anti-K or anti-$Fy^a$ "... has yet to be obtained by this process."

DE—A—2 614 865 (equivalent to US—A—4 046 871) discloses serological albumin compositions. The essential component of the compositions is polymerised bovine serum albumin, which contains from two to about eight more monomeric bovine albumin units which are joined to each other through a covalent peptide band.

According to the present invention, there is provided a method for determining the presence or absence on the red cells of a given individual of D antigen which comprises the steps of: ·

(a) obtaining a sample of red blood cells to be tested.

(b) adding to and mixing with said sample an antibody reagent maintained at a pH of between 7.5 and 8.3 and comprising reduced and S-alkylated IgG antibody to the D antigen which has an average titer of at least 32 for D cells in the slide or rapid tube test to form a mixture; and

(c) observing said mixture to determine whether agglutination does or does not occur; characterised in that:

(d) the reagent comprises polymerised bovine albumin to give a total protein concentration of from 6 to 10% by weight; and

(e) the observation is carried out without substantial incubation, whereby the determination is carried out rapidly.

According to a second aspect of the present invention, there is provided an antibody reagent, useful for rapidly determining the presence or absence on red blood cells of D antigen, comprising reduced, S-alkylated IgG antibody to the D antigen having in the slide test or rapid tube test an average titer of at least 32 for D cells maintained at a pH of between 7.5 and 8.3 by an effective buffering amount of a compatible buffer, characterised in that: the antibody is maintained in a medium containing polymerised bovine albumin to give a total protein concentration of from 6 to 10% by weight.

The present invention also includes a method for making a reagent according to the invention.

The present invention provides a rapid method for detecting $Rh_0$ D antigen (which is in the Rh blood group system), and reagents for practicing this method which remove the disadvantages attendant on the prior art methods. The subject antibody reagent allows for the first time the use of IgG antibody in a low-protein slide or rapid tube test. The subject test has no nonspecific reaction with IgG coated cells due to the low-protein content. Moreover, the subject Rh antibody reagents meet and exceed FDA standards for potency and specificity for the slide test or rapid tube test. These FDA standards are contained in Subpart C of Part 660, Subchapter F of the Federal Food, Drug and Cosmetic Act (see 42 Federal Regulations 54542 ff, No. 195, October 7, 1977).

The anti-D reagent is generally prepared by the following procedure. Serum is obtained from individuals having high levels of anti-D IgG in their bloodstream and is treated to remove certain contaminants and purify the IgG. While the anti-specific method of producing the anti-D antiserum for use in preparing the subject reagent is not part of the present invention, one suitable process is the following. Other processes for producing suitable anti-D antisera are known.

If necessary, the plasma is defibrinated with an appropriate amount of bovine thrombin; decalcified with sodium oxalate; clarified by centrifugation; and neutralized by the addition of blood group specific substance A and/or AB.

Lipids are substantially removed from the serum by extraction with trichlorotrifluoroethane. The serum is heated to a maximum of 56.6°C and then cooled to 2—8°C, after which trichlorotrifluoroethane is blended in at high speeds until the serum foams. After the serum has been stored at 2—9°C until the foam dissipates, it is centrifuged, heated again, and stored at 2—8°C. The pH is adjusted to 7.3—8.3 and the serum is stored at −20°C or below.

This substantially lipid-free serum may then be treated in the following manner to produce the subject reagent.

The pH of the substantially lipid-free serum is adjusted to about 8.6 using an effective amount of a compatible buffer such as 2M tris-(hydroxymethyl)aminomethane (TRIS). One volume of 0.04M dithiothreitol in saline is added to about four volumes of the buffered serum and the mixture is allowed to stand at about 20—30°C for a minimum of about thirty minutes.

Following this treatment, one volume of 0.4M iodoacetamide in distilled water is added to about four volumes of the treated serum. This mixture is allowed to stand at about 20—30°C for a minimum of about sixty minutes. The resulting serum is then dialyzed twice at 2—8°C using ten volumes of freshly prepared 0.02M TRIS buffer in 0.13M NaCl at pH 7.5—8.3 and preferably pH 7.7—8.1 each time. After the second dialysis, sodium azide is added to the treated serum to a final concentration of 0.1% as a preservative, and the total protein concentration is adjusted to about 6—10 weight percent with polymerised bovine albumin. Polymerised bovine serum albumin is preferably used. If necessary, the concentration of antibody in the subject antibody reagent may be adjusted by dilution with 0.02M TRIS buffer in 0.13M NaCl containing 6—8 weight percent polymerised bovine serum albumin.

While one particular process for producing the subject reagent has been given above, it is contemplated that other suitable reducing agents may be substituted for the preferred dithiothreitol used therein; such reducing agents include, for example, 2-mercaptoethanol and dithioerythritol. While odoacetamide is used above as the S-alkylating agent, it is clearly contemplated that other alkylating agents may be used, for example, iodoactic acid. Alternately, equivalent methods of preventing reformation of the disulfide bond may be used.

The pH range of 7.5—8.3 and preferably 7.7—8.1 is important to the proper functioning of the subject reagent. Outside the 7.5—8.3 pH range, the reactions of the subjet antibody reagents tend to be somewhat weaker. While this pH range may be maintained by an effective buffering amount of any compatible bufer (i.e., one which has no adverse effect on the antibodies or the red blood cells) TRIS buffer is preferred.

The present invention provides improved method of rapidly determining the presence or absence of the antigen on red blood cells of a given individual using the reagent described above. It is anticipated that any of the prior art rapid typing methods, such as the slide test, modified ("rapid") tube test, or stick test method, may be employed, but substituting the subject reagent for the prior art IgG antiserum used therein. While directions for use of the subject antibody reagent in certain prior art tests are given below, it is anticipated that the use of the subject antibody reagent is not limited to those specific tests but may be used generally in any method which comprises the steps of:

A) Obtaining a sample of red blood cells to be tested;
B) Adding to and mixing with said sample the subject antibody reagent to form a mixture; and
C) Without substantial incubation observing said mixture to determine whether agglutination does or does not occur.

The term "without substantial incubation" as used herein is intended to mean that a delay of only 2—15 minutes occurs prior to the observing step. This is in contrast to the description the Romans, et al., article where a substantial incubation step occurs. In many instances the observing step may take place "without incubation", by which is meant that no deliberate delay occurs between the mixing step and the observing step. The observing step can take place without incubation in the slide test and in the modified tube method. For the other methods, an incubation of about two months is desirable to obtain a high degree of agglutination.

The modified tube method may be conducted as follows. First, a suspension of the red blood cells to be tested is prepared. Conveniently, this may be a five percent suspension in normal group compatible serum, in the patient's own serum or plasma, or in isotonic saline.

Second, a small amount (conveniently one drop) of the subject antibody reagent is mixed with an equal amount of the red blood cell suspension in a small test tube, after which the tube is centrifuged without prior incubation to produce a "button" of red cells and a clear supernate, as known in the art.

Finally, the presence or absence of agglutination is determined for example by resuspending the cells by gentle agitation and macroscopically examining them. If desired, a control of saline or nonspecific protein (e.g., 6—8% bovine albumin) in saline may be employed to aid in this determination.

A slide test may be performed as follows. First, a suspension of the red blood cells to be tested is again prepared in normal group compatible serum or in the patient's own serum or plasma. Whole blood may be used without dilution, since the concentration of red blood cells in the test sample conveniently should be about 40—50%.

Second, a small amount (e.g., about one drop) of the subject antibody reagent is mixed with about double the volume of the cell suspension on a prewarmed glass slide. Preferably, the temperature of the slide is about 40—45°C, although ambient temperatures (e.g., 20—30°C) may be used.

Finally, after the red blood cell suspension and the antibody reagent have been thoroughly mixed but without further incubation, the presence or absence of agglutination is evaluated by tilting the slide back and forth for several minutes and observing for any agglutination. A control of saline or nonspecific protein (e.g., 6—8% bovine albumin) in saline may also be subjected to the same series of steps in place of the subject antibody reagent for use as a negative control.

Negative results for D antigen on any of these tests may be confirmed by the $D^u$ test as follows. To a small amount of the five percent suspenson of red blood cells referred to above is added an equivalent amount of the subject anti-D antibody reagent with mixing. After incubation of this mixture at about 37°C for about 15 minutes, the cells are washed, preferably three times with isotonic saline, and anti-human serum is added to the washed cells. The cells are then centrifuged as described above and the presence or absence of agglutination is determined by observation. A positive result on this test (but negative on the D test and control) indicates Rh positive cells of the $D^u$ variety. A saline suspension of the same red blood cells (without antibody reagent) is used as a control.

While these methods of use of the subject antibody reagent have been given by way of illustration, it is anticipated that the subject Rh reagents are suitable for use in any agglutination test for detection of the D antigen and especially for use in such tests which do not require incubation.

The present invention will be further described with reference to the following Figures.

Detailed Description of the Drawings
Figure 1 shows a comparison of a lot of the subject anti-D reagent with commercial anti-D serum for saline tube test;
Figure 2 shows a comparison of a lot of the subject anti-D reagent with commercial anti-D serum for slide and modified tube test, in which both reagents are used to perform a modified tube test.

Figure 3 shows the same comparison as Figure 2 in which the reagents are reacted with serum suspended test cells;

Figure 4 illustrates the same comparison as Figures 2 and 3, but for the stick test;

Figure 5 illustrates the same comparison for a slide test;

Figure 6 illustrates a comparison of the subject anti-D reagent with an anti-D reagent containing normal bovine albumin.

The performance of the subject antibody reagents has been demonstrated in tests which compared them to current reagents for slide and modified tube tests and for saline tube test, respectively. As shown by these tests, the subject antibody reagents perform at least as well as current reagents, and, because of their unique characteristics, offer significant advantages.

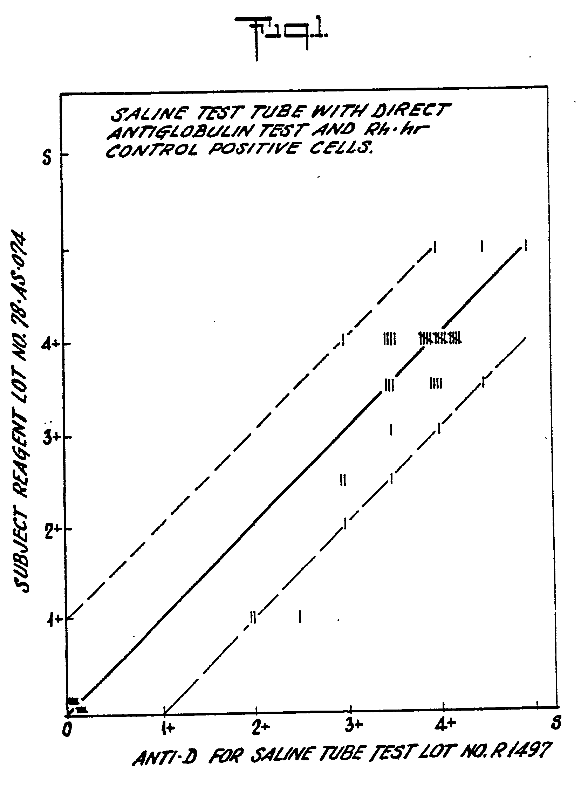

The specificity of the subject anti-D antibody reagent Lot 78-AS-074 was demonstrated in comparison with Ortho anti-D serum for saline tube test Lot R1497. For this test, 284 direct antiglobulin test positive red blood cells (as determined with polyspecific anti-human serum) were selected. These direct antiglobulin test positive cells were selected because they are likely to spontaneously agglutinate in a high protein medium such as that used in prior art IgG antibody reagents. Hence they provide a good panel of cells for demonstration of the advantages of the subject anti-D antibody reagent, which has a relatively low protein concentration. Of these cells, 55 reacted with Ortho Rh-hr Control (reagent solution without antibody), which would invalidate test results obtained with a high protein, high viscosity reagent such as anti-D serum for slide and modified tube test. Hence, these 55 cells could be successfully tested using the subject anti-D antibody reagent but not the prior art high protein IgG anti-D antibody reagent. Three of the 55 cells agglutinated with saline and could not be tested by routine procedures. The remaining 52 cells were tested according to the saline tube test procedures recommended in the labelling. The results are graphically depicted in Figure 1. Ten cells exhibited negative reactions with both reagents. Forty-two cells exhibited positive reactions of comparable strength, with both reagents. From these results, it can be concluded that the subject anti-D antibody reagent is as specific as Ortho anti-D serum for saline tube test, and that it can be used in tests where Ortho anti-D serum for slide and modified tube test gives nonspecific reactions.

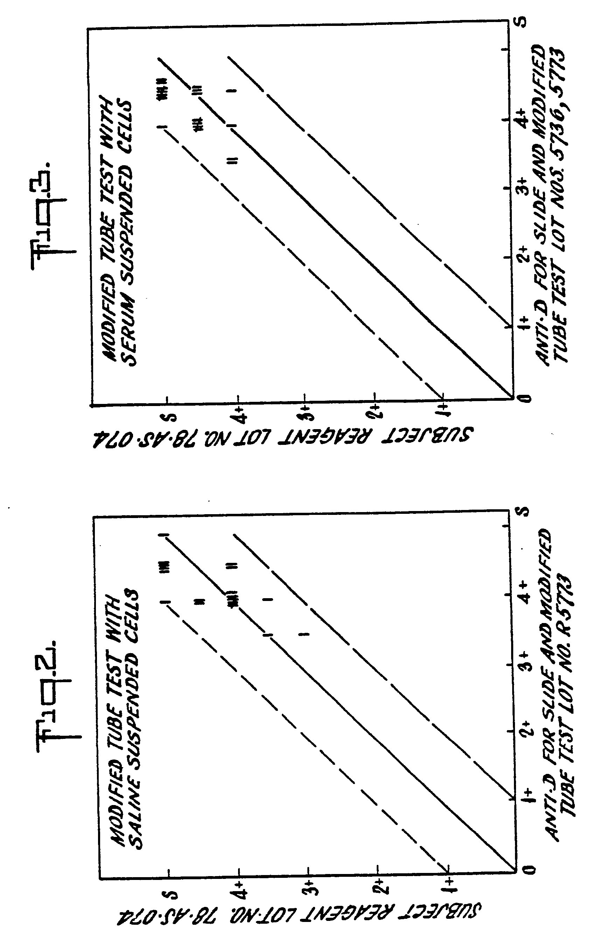

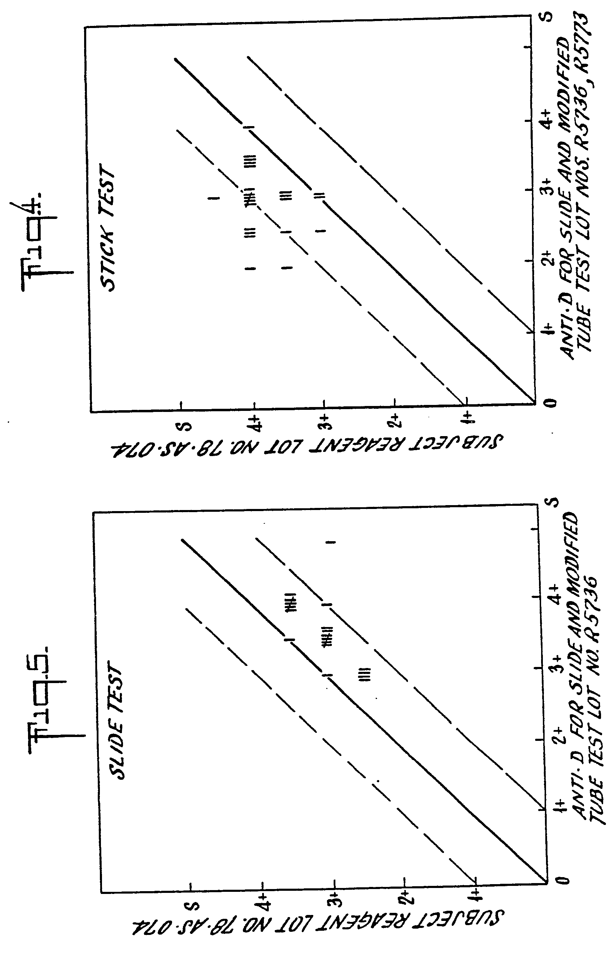

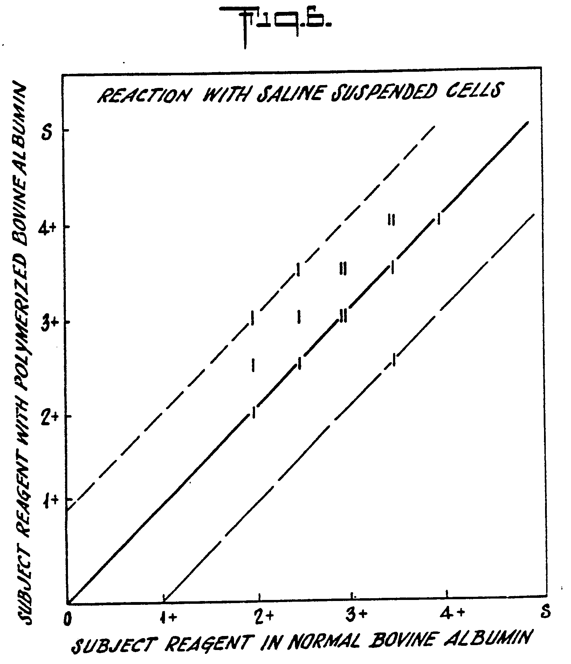

The subject anti-D antibody reagent Lot 78-AS-074 was compared with ortho anti-D serum for slide and modified tube test, Lots R5736 and R5773, by performing modified tube tests, stick tests and slide tests according to the procedures recommended in the labelling. The modified tube tests were performed using saline suspended test cells and serum suspended test cells, respectively. The results obtained with the saline suspended test cells appear in Figure 2, and indicate that the reactions with two antisera are comparable. The results obtained with the serum suspended test cells appear in Figure 3. From these results, the reactions with the subject anti-D antibody reagent appear to be slightly stronger than the reactions with anti-D serum for slide and modified tube test. The stick test reactions depicted in Figure 4 indicate that reactions with the subject anti-D antibody reagent are stronger. The results of the slide test appear in Figure 5. In these tests the subject anti-D antibody reagent exhibited slightly weaker reactions than anti-D serum for slide and modified tube test. The use of polymerized bovine serum albumin (Figure 6) improves the strength of the agglutination. The Figure shows the strength of agglutination for each composition with two or three week old red cells (3—5%) suspended in saline.

In Figures 1—6, the degree of agglutination is represented by the designations 0, $1^-$, $2^-$, $3^-$, $4^-$, and S, in which 0 designated no observable agglutination, $1^-$ through $4^-$ designate progressively greater degrees of agglutination, and S designates "solid" or maximum agglutination.

## Claims

1. A method for determining the presence or absence on the red cells of a given individual of D antigen which comprises the steps of;

(a) obtaining a sample of red blood cells to be tested;

(b) adding to and mixing with said sample an antibody reagent maintained at a pH of between 7.5 and 8.3 and comprising reduced and S-alkylated IgG antibody to the D antigen which has an average titer of at least 32 for D cells in the slide or rapid tube test to form a mixture; and

(c) observing said mixture to determine whether agglutination does or does not occur; characterised in that:

(d) the reagent comprises polymerised bovine albumin to give a total protein concentration of from 6 to 10% by weight; and

(e) the observation is carried out without substantial incubation, whereby the determination is carried out rapidly.

2. The method of claim 1, which is a slide test method wherein the adding and mixing step (b) is carried out on a test slide.

3. The method of claim 2, wherein the test slide is heated to 40 to 45°C prior to the adding and mixing step (b).

4. An antibody reagent, useful for rapidly determining the presence or absence on red blood cells of D antigen comprising reduced, S-alkylated IgG antibody to the D antigen having in the slide test or rapid tube test an average titer of at least 32 for D cells maintained at a pH of between 7.5 and 8.3 by an effective buffering amount of a compatible buffer, characterised in that: the antibody is maintained in a medium containing polymerised bovine albumin togive a total protein concentration of from 6 to 10% by weight.

5. A method for preparing an antibody reagent useful for rapidly determining the presence or absence on red blood cells of D antigen, which reagent comprises IgG antibody having in the slide test or rapid tube test an average titer of at least 32 for D cells, which method comprises the steps of:

(a) obtaining a substantially lipid-free human antiserum containing IgG antibody to the D antigen;

(b) adjusting the pH of said antiserum to about 8.6 by addition of an effective buffering amount of a compatible buffer;

(c) reducing said buffered antiserum with a reducing agent;

(d) S-alkylating said reduced buffered antiserum with S-alklating agent; and

(e) dialysing said S-alkylated reduced antiserum at a pH between 7.5 and 8.3 characterised in that;

(f) the total protein concentration of the dialysed antiserum is adjusted to from 6 to 10% by weight by the addition of polymerised bovine albumin.

6. The method of claim 5, wherein the buffer used in the step (b) is 2M tris-(hydroxymethyl) aminomethane.

7. The method of claim 5 or claim 6, wherein the reducing step (c) is accomplished by mixing one volume of 0.04M dithiothreitol in saline with four volumes of the buffered antiserum from step (b) and allowing the mixture to stand at 20 to 30° for at least 30 minutes.

8. The method of any one of claims 5 to 7, wherein the S-alkylating step (d) is accomplished by mixing one volume of 4M iodoacetamide in distilled water with four volumes of the reduced antiserum from step (c) and allowing the mixture to stand at 20 to 30°C for at least sixty minutes.

9. The method of any one of claims 5 to 8, wherein the dialysing step (e) is accomplished by twice dialysing the S-alkylated antiserum from step (d) at 2 to 8°C using ten volumes to 0.02M tris-(hydroxymethyl) aminomethane in 0.13M NaCl at a pH of from 7.5 to 8.3.

10. The method of claim 9, wherein the pH of the dialysis buffer is from 7.7. to 8.1

## Patentansprüche

1. Verfahren zur Bestimmung der Anwesenheit oder Abwesenheit von D-Antigen auf den roten Zellen eines gegebenen Individuums, umfassend die Stufen:

(a) Bereitstellen, einer Probe zu prüfender roter Blutzellen,

(b) Zusetzen zu und Vermischen mit der Probe eines bei einem pH zwischen 7,5 und 8,3 gehaltenen Antikörper-Reagenz, umfassend reduzierte und S-akylierte Ig G-Antikörper bezüglich dem D-Antigen, die einen Durchschnittstiter von mindestens 32 für D-Zellen gemäß dem Objektträger-Test oder Röhrchen-Schnelltest aufweisen, um eine Mischung zu bilden und

(c) Beobachten der Mischung, um festzustellen, ob Agglutination auftritt oder nicht auftritt, dadurch gekennzeichnet, daß

(d) das Reagenz polymerisiertes Rinderalbumin, um eine Gesamtproteinkonzentration von 6 bis 10 Gew.-% zu ergeben, aufweist und

(e) die Beobachtung ohne wesentliche Inkubation ausgeführt wird, wodurch die Bestimmung schnell durchgeführt wird.

2. Verfahren nach Anspruch 1, wobei es sich um eine Objektträger-Testmethode handelt, wobei die Zusetz- und Mischstufe (b) auf einem Testobjektträger durchgeführt wird.

3. Verfahren nach Anspruch 2, wobei der Testobjektträger vor der Zusetz- und Mischstufe (b) auf 40 bis 45°C erwärmt wird.

4. Antikörper-Reagenz, das zur schnellen Bestimmung der Anwesenheit oder Abwesenheit von D-Antigen auf roten Blutzellen geeignet ist, umfassend reduzierte, S-alkylierte IgG-Antikörper bezüglich dem D-Antigen, die beim Objektträger-Test oder Röhrchen-Schnelltest einen Durchschnittstiter von mindestens 32 für D-Zellen aufweisen, das mittels einer wirksamen Puffermenge eines verträglichen Puffers bei einem pH zwischen 7,5 und 8,3 gehalten wird, dadurch gekennzeichnet, daß die Antikörper in einem Medium gehalten, werden, das polymerisiertes Rinderalbumin, um eine Gesamtproteinkonzentration von 6 bis 10 Gew.% zu ergeben, enthält.

5. Verfahren zur Herstellung eines Antikörper-Reagenz, das zur schnellen Bestimmung der Anwesenheit oder Abwesenheit oder Abwesenheit von D-Antigen auf roten Blutzellen geeignet ist, umfaßend IgG-Antikörper, die beim Objektträger-Test oder Röhrchen-Schnelltest einen Durchschnittstiter von mindestens 32 für D-Zellen aufweisen, wobei das Verfahren folgende Stufen umfaßt:

(a) Bereitstellen eines im wesentlichen lipoidfreien Human-Antiserums, enthaltend IgG-Antikörper bezüglich dem D-Antigen,

(b) Einstellen des Ph des Antiserums auf etwa 8,6 durch Zugabe einer wirksamen Puffermenge eines verträglichen Puffers,

(c) Reduzieren des gepufferten Antiserums mit einem Reduktionsmittel,

(d) S-Alkylieren des reduzierten, gepufferten Antiserums mit einem S-Alkylierungsmittel und

(e) Dialysieren des S-alkylierten, reduzierten Antiserums bei einem pH zwischen 7,5 und 8,3, dadurch gekennzeichnet, daß

(f) die Gesamtproteinkonzentration des dialysierten Antiserums durch Zugabe von polymerisiertem Rinderalbumin auf 6 bis 10 Gew.% eingestellt wird.

6. Verfarhen nach Anspruch 5, wobei in der Stufe (b) als Puffer 2-M-Tris(hydroxymethyl)-aminomethan verwendet wird.

7. Verfahren nach Anspruch 5 oder 6, wobie die Reaktionsstufe (c) in der Weise durchgeführt wird, daß man ein Volumenteil 0,04-M-Dithiothreit in Salzlösung mit 4 Volumenteilen des gepufferten Antiserums aus Stufe (b) vermischt und die Mischung mindestens 30 min bei 20 bis 30°C stehenläßt.

8. Verfahren nach einem der Ansprüche 5 bis 7, wobei die S-Alkylierungsstufe (d) in der Weise durchgeführt wird, daß man ein Volumenteil 4M-Jodacetamid in destilliertem Wasser mit 4 Volumenteilen des reduzierten Antiserums aus Stufe (c) vermischt und die Mischung mindestens 60 min bei 20 bis 30°C stehenläßt.

9. Verfahren nach einem der Ansprüche 5 bis 8, wobei die Dialysierstufe (e) in der Weise durchgeführt wird, daß man das S-alkylierte Antiserum aus Stufe (d) bei 2 bis 8°C unter Verwendung von 10 Volumenteilen 0,02M-Tris(hydroxymethyl)-aminomethan in 0,13M-NaCl bei einem pH von 7,5 bis 8,3 zweimal dialysiert.

10. Verfahren nach Anspruch 9, wobei der pH des Dialysepuffers 7,7 bis 8,1 beträgt.

**Revendications**

1. Procédé pour la détermination de la présence ou de l'absence sur des globules rouges d'un individu donné d'antigène D qui comprend les étapes de:

(a) obtenir un échantillon de globules rouges à tester;

(b) ajouter et mélanger audit échantillon un réactif anticorps maintenu à un pH compris entre 7,5 et 8,3, et comprenant un anticorps IgG S-alkylé de l'antigène D qui a un titre moyen d'au moins 32 pour les cellules D dans un test sur lamelle ou sur tube rapide pour former un mélange; et

(c) observer ledit mélange pour déterminer si l'agglutination se produit ou non; caractérisé en ce que:

(d) le réactif comprend de l'albumine bovine polymérisée pour donner une concentration totale en protéine de 6 à 10% en poids; et

(e) l'observation est effectuée sans incubation substantielle, par quoi la détermination est effectuée rapidement.

2. Procédé selon la revendication 1, qui est un procédé de test sur lamelle où l'étape d'addition et de mélange (b) est effectuée sur une lamelle.

3. Procédé selon la revendication 2, où la lamelle est chauffée à 40 à 45°C préalablement à l'étape d'addition et de mélange (b).

4. Réactif anticorps, utile pour la détermination rapide de la présence ou de l'absence sur des globules rouges sanguins d'antigène D comprenant un anticorps IgG S-alkylé réduit de l'antigène D ayant dans le test sur lamelle ou le test sur tube rapide un titre moyen d'au moins 32 pour les cellules D maintenu à un pH compris entre 7,5 et 8,3 par une quantité efficace de tampon d'un tampon compatible, caractérisé en ce que: l'anticorps est maintenu dans un milieu contenant de l'albumine bovine polymérisée pour donner une concentration totale en protéine de 6 à 10% en poids.

5. Procédé pour préparer un réactif anticorps utile pour déterminer rapidement la présence ou l'absence sur des globules rouges sanguins d'antigène D, lequel réactif comprend un anticorps IgG ayant dans le test sur lamelle ou dans le test sur tube rapide un titre moyen d'au moins 32 pour les cellules D, ledit procédé comprenant les étapes de:

(a) obtenir un antisérum hamain substantiellement exempt de lipides contenant l'anticorps IgG de l'antigène D;

(b) adjuster le pH dudit antisérum à environ 8,6 par addition d'une quantité efficace de tampon d'un tampon compatible;

(c) réduire l'antisérum tamponné précité avec un agent réducteur;

(d) S-alkyler ledit antisérum tamponné réduit ave l'agent S-alkylant; et

(e) dialyser ledit antisérum réduit S-alkylé à un pH compris entre 7,5 et 8,3, caractérisé en ce que;

(f) la concentration totale en protéine de l'antisérum dialysé est adjustée de 6 à 10% en poids par addition d'albumine bovine polymérisée.

6. Procédé selon la revendication 5, où le tampon utilisé dans l'étape (b) est du tris-(hydroxyméthyl)aminométhane 2M.

7. Procédé selon la revendication 5 ou 6, où l'étape de réduction (c) est accomplie en mélangeant un volume de dithiothréitol 0,04M en solution saline avec quatre volumes d'antisérum tamponné de l'étape (b) et en maintenant le mélange à 20 à 30°C pendant au moins 30 minutes.

8. Procédé selon l'une des revendications 5 à 7, où l'étape de S-alkylation (d) est accomplie en mélangeant un volume d'iodoacétamide 4M dans de l'eau distillée avec quatre volumes de l'antisérum réduit de l'étape (c) et en maintenant le mélange à 20 à 30°C pendant au moins 60 minutes.

9. Procédé selon l'une des revendications 5 à 8, où l'étape de dialyse (e) est accomplie en dialysant deux fois l'antisérum S-alkylé de l'étape (d) à 2 à 8°C en utilisant dix volumes de tris-(hydroxyméthyl)aminométhane 0,02M dans NaCl 0,13M à un pH de 7,5 à 8,3.

10. Procédé selon la revendication 9, où le pH du tampon de dialyse est de 7,7 à 8,1.

## Fig 1.

SALINE TEST TUBE WITH DIRECT ANTIGLOBULIN TEST AND Rh·hr CONTROL POSITIVE CELLS.

Y-axis: SUBJECT REAGENT LOT NO. 78·AS·074

X-axis: ANTI·D FOR SALINE TUBE TEST LOT NO. R 1497

Fig.2.

MODIFIED TUBE TEST WITH
SALINE SUSPENDED CELLS

Fig.3.

MODIFIED TUBE TEST WITH
SERUM SUSPENDED CELLS

(Fig. 2) Y-axis: SUBJECT REAGENT LOT NO. 78·AS·074; X-axis: ANTI·D FOR SLIDE AND MODIFIED TUBE TEST LOT NO. R5773

(Fig. 3) Y-axis: SUBJECT REAGENT LOT NO. 78·AS·074; X-axis: ANTI·D FOR SLIDE AND MODIFIED TUBE TEST LOT NOS. 5736, 5773

EP 0 022 669 B2

Fig.5.

SLIDE TEST

Fig.4.

STICK TEST

EP 0 022 669 B2

Fig.6.

REACTION WITH SALINE SUSPENDED CELLS